(19) Europäisches Patentamt
European Patent Office
Office européen des brevets

(11) Publication number: 0 129 382
B1

(12) # EUROPEAN PATENT SPECIFICATION

(45) Date of publication of patent specification: 26.09.90

(51) Int. Cl.$^5$: **A 61 K 31/545**, A 61 K 9/52

(21) Application number: 84303908.2

(22) Date of filing: 08.06.84

(54) **Long-acting formulation of cefaclor and its preparation.**

(30) Priority: 15.06.83 JP 108289/83

(43) Date of publication of application:
27.12.84 Bulletin 84/52

(45) Publication of the grant of the patent:
26.09.90 Bulletin 90/39

(84) Designated Contracting States:
AT BE CH DE FR LI LU NL SE

(56) References cited:
GB-A- 715 305
GB-A-2 103 486
US-A-3 906 086
US-A-4 250 166

(73) Proprietor: SHIONOGI & CO., LTD.
1-8, Doshomachi 3-chome Chuo-ku
Osaka 541 (JP)

(72) Inventor: Sakamoto, Teruo
11-10, Nagisasakae-machi
Hirakata-shi Osaka (JP)
Inventor: Kawai, Sadao
15-7, 1-chome, Kawai
Matsubara-shi Osaka (JP)
Inventor: Noda, Kinzaburo
418, 3-chome, Ikejiri
Itami-shi Hyogo (JP)
Inventor: Takeda, Toyohiko
44-8, Sumiyoshidai Higashinada-ku
Kobe-shi Hyogo (JP)
Inventor: Kato, Hiroshi
8-15, Moniwahitokitayama
Sendai-shi Miyagi (JP)

(74) Representative: Hardisty, David Robert et al
BOULT, WADE & TENNANT 27 Furnival Street
London EC4A IPQ (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 129 382 B1

Courier Press, Leamington Spa, England.

**Description**

The present invention provides oral long-acting formulations of a cephalosporin-type antibiotic, cefaclor [3 - chloro - 7 - D - (2 - phenylglycinamido) - 3 - cephem - 4 - carboxylic acid monohydrate] (hereinafter referred to as CCL).

CCL was developed in the U.S.A. as an orally administrable cephalosporin antibiotic which has a broader spectrum and is 2 to 8 times more active than an analogue, cefalexin (hereinafter referred to as CEX), in in vitro antimicrobial tests. The antibiotic CCL has ordinarily been applied in the treatment of a variety of infections since it has more potent bactericidal action than CEX and is highly effective in clinical use. The formulation of CCL presently marketed, however, must be administered 3 times a day, i.e., at intervals of eight hours, and the time of administration is not necessarily in accordance with ordinary mealtimes, and it is naturally desired to remove such disadvantages. L-Keflex® (Lilly's brand of long-acting CEX formulation) is representative of the long-acting oral antibiotic formulations which has been marketed [disclosed in U.S. Patent No. 4250166 (Japanese Patent Publication No. 55-47611)].

The present invention provides a long-acting formulation of CCL comprising a mixture of a non-enteric coated component of cefaclor to serve as a rapid-release component with an enteric coated component of cefeclor to serve as a slow-release component at a ratio of about 4:6 respectively based on potency of cefaclor wherein said non-enteric coated component contains at least one additive selected from lactose, sucrose, D-mannitol, corn starch, wheat starch and low-substituted hydroxypropylcellulose in an amount of up to 75% weight based on the whole non-enteric coated component and said enteric coated component contains at least one additive selected from lactose, sucrose, D-mannitol, corn starch, wheat starch and low-substituted hydroxypropylcellulose in an amount of up to 75% weight based on the whole enteric-coated component, and an enteric-coating which is a film soluble in the pH range 5.0 to 7.0.

The rapid-release component is formulated to give a maximum blood level of CCL rapidly and the slow release component to give a maximum blood level within a period of 3 to 7 hours after administration.

The invention will now be further described and illustrated, in part by reference to the accompanying drawings, in which:—

Fig. 1 shows time-dependent curves for mean blood-level when CCL or CEX was administered at a single dose of 259 mg to some volunteers after fasting; the ordinate and abscissa show the drug concentrations (μg/ml) and the time (hours), respectively;

Fig. 2 is two charts (A and B) which show the differences in both drug concentrations (ordinate) and the period of time (abscissa) needed for 99% sterilization, respectively, of *Escherichia coli* NIH JC2 or *Escherichia coli* No. 29, comparing CEX and CCL;

Fig. 3 shows curves for the viable cells versus time relationship in various cases where the simulated blood CCL level responding to the respective combination ratio (rapid:slow) of a long-acting CCL formulation had an influence on the viable cell numbers of *Staphylococcus aureus* No. 1307 in a culture medium; the ordinate and abscissa show the percentage of the viable cell number based upon the inoculum size of the test microorganism and time (hours), respectively; and

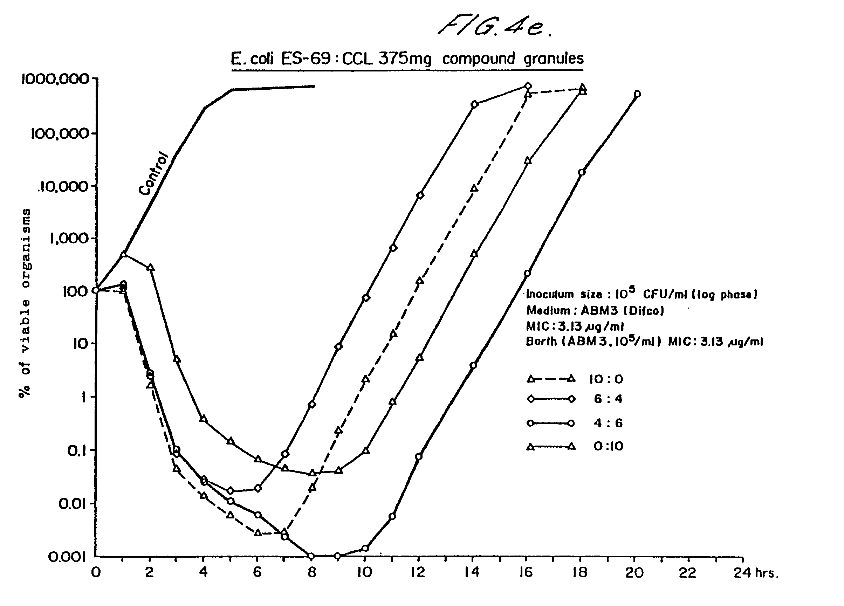

Figs. 4(a) to (e) show curves of the viable cell number versus time relationship at various combination ratios with respect to *Staphylococcus aureus* No. 1021, *Staphylococcus aureus* No. 6, *Escherichia coli* ES-80, *Escherichia coli* ES-68, *Escherichia coli* ES-69, respectively, as in Fig. 3; the ordinate and abscissa show the percentage of the viable cell number based upon the inoculum size of the test microorganisms and time (hours), respectively.

The present invention relates to a long-acting formulation of CCL (hereinafter referred to as long-acting CCL), more particularly, it relates to long-acting CCL which comprises a non-enteric coated CCL component as a rapid release component and an enteric coated CCL component as a slow release component, the former component being formulated so as to release the active ingredient substantially immediately after administration and the latter being formulated e.g., into a film-coated formulation covered with an enteric-coating film soluble at pH 5.0 to 7.0, preferably at pH 5.5 to 6.5.

As mentioned above, the ordinary formulation of CCL requires administration every eight hours to give the best efficacy. It is, however, usually administered after meals because administration precisely at 8 hour intervals is in practice inconvenient.

In many cases, normal meals three times a day are usually taken in the daytime and there is, therefore, an intermission of about 12 hours from an evening meal (supper) to the next meal in the morning (breakfast). Furthermore, administration every 8 hours fixed independent of mealtimes causes trouble for outpatients since they must keep in mind the exact time for administration without fail.

For patients with slight or moderate cases of infection, to whom the ordinary formulations of CCL have mainly been administered, it is desired to provide a special formulation of CCL for which the frequency of administration is reduced so that the drug can be taken at the prefixed time safely, because such patients are not always required to stay in a hospital, e.g., they often have to take the formulation at school or in an office.

The present inventors have attempted to develop a special formulation of CCL by which the problem of failure of administration is avoided, which may be taken at unified intervals, and which affords the same clinical efficacy at the same daily dose as the prior art ordinary formulations. The present invention is based upon these considerations. In other words, taking into consideration the fact that the duration between breakfast and supper is usually about 12 hours for most people, it is naturally appropriate to take the drug twice a day after each breakfast and supper. Thus, it is desirable to provide the drug in a form which enables one to regulate the conditions of administration, i.e., by taking the drug after breakfast and supper, and the taking of the drug at equal intervals helps avoid a failure of administration and a decrease of clinical efficacy caused by a time lag in administration. In addition, the inconvenience imposed on patients who have always to carry the drug could thus be avoided.

The present inventors have studied the in vivo distribution of CCL (blood level) and the in vitro antibacterial activity.

Fig. 1 shows the blood levels versus time curves when CCL or CEX is administered at a single dose of 250 mg to some volunteers after fasting (details of the tests are given below). The absorption and the excretion of both drugs resemble each other in the pattern of the respective curves, but the maximum blood level is lower and the half-life shorter in CCL than in CEX.

Additionally, the bactericidal effects of CCL were compared with those of CEX by the following in vitro tests (details of the tests are given below). The concentrations of CCL or CEX and the time which are needed for diminishing by 99% the inoculum size were measured (Fig. 2, Table 2). As can be seen from the results of the tests, CEX needs at least one MIC (concentration of CEX corresponding to MIC) for 99% diminution of the inoculum size; in contrast, CCL sterilizes 99% of the microorganisms even 1/2 MIC within a shorter period of time than CEX.

Thus, it was confirmed that the characteristics of CCL were quite different from those of CEX with respect to the concentration and time needed for sterilization as well as the absorption and excretion characteristics.

The present inventors attempted to make formulations consisting of rapid-release CCL components and slow-release CCL components with a view to maintaining the blood level of CCL at over a certain level for a long period of time. As a result, they discovered the optimum combination ratio of the rapid-release CCL component to the slow-release CCL component (total amount of CCL in the formulation may be 375 mg by potency). The invention is based upon this work and the aforesaid discovery. The work was performed as follows.

1. A rapid-release and a slow-release component were independently prepared.

2. The rapid-release component (375 mg as CCL) was administered to a few volunteers 30 minutes after a meal and the average blood level determined from the actual values.

3. In the same manner as in item 2, the slow-release component (375 mg as CCL) was tested.

4. The blood level for various combination ratios (rapid component:slow component) was proportionally calculated from the results in items 2. and 3. using all integral ratios of the rapid-release component to the slow-release component based upon CCL potency, from 1:9 to 9:1.

5. Time-dependent blood level changes for all combinations (rapid:slow=10:0 to 0:10) were simulated in a culture medium, in which the microorganisms were brought into contact with CCL at concentrations changing time-dependently, and changes in the viable cell numbers were observed. The results obtained through the above procedures are illustrated in Fig. 3.

In this test, *Staphylococcus aureus* No. 1037 was chosen as a representative of gram positive bacteria. The respective growth curves in Fig. 3 show a similar tendency concerning:

a. the rate of decrease of the viable cells (i.e., slopes of the curves in the region A), and

b. the growth-rate after the bottom (slopes of the curves in the region B), the considerable differences are observed amongst the respective curves concerning:

i. the time at which the number of the viable cells began decreasing,

ii. the time at which the cells began to revive, and

iii. the number of viable cells at the time of cell revival occurring.

In this connection, it can be recognized that the 4:6 formulation (the combination ratio based upon potency of CCL) gave the most satisfactory result with respect to the above items i to iii.

Although one object of this invention is to provide the optimum combination ratio of CCL between the two components; it should be noted that the term "long-acting formulation of CCL" used herein means any formulation composed of rapid-release and slow-release components which keeps the activity of CCL for a longer period of time than ordinary formulations of CCL do.

The present long-acting formulations of CCL may be prepared into multi-layer formulations consisting of rapid-release and slow-release portions, or prepared as blended formulations which are a mixture of the two independently prepared components in the desired ratio. Additionally, the dosage form used is not limited specifically to any particular form, and any form may be applied to this invention if it is a conventional form for orally administrable antibiotics. The multi-layer formulations may be formed into granules or beads, which may further be encapsulated to provide capsules or formed into tablets according to conventional procedures.

Blended formulations include powders, granules, beads, capsules and tablets amongst the possible forms.

In this invention, the rapid-release portion (or component) is generally a plain formulation of CCL to which no treatment for retardation nor enteric coating-film has been given. Thus, the rapid-release portion may be e.g., unprocessed CCL powder, or granules, beads or tablets prepared in a conventional manner.

The slow-release portion (or component) is generally in the form of microcapsules prepared from unprocessed CCL powder by film-coating, or enteric coated formulations prepared from granules or beads as mentioned above. The slow-release portion plays an important role in the retardation of CCL in this invention; the slow-release portion (or component) may be prepared as explained below in more detail.

Since the main absorptive organ for CCL is the upper part of small intestine (as with CEX) it is necessary to prepare the slow-release portion so that it completely and rapidly releases CCL within a pH range of about 5.0 to 7.0 in order to increase the absorption rate of CCL. Additionally, since the slow-release portion is, in this invention, formulated into a long-acting formulation in combination with a rapid-release portion, it is necessary for the slow-release portion to be insoluble in acidic media, i.e., in the stomach. In this connection, the aforementioned problems may be resolved by coating a desirably soluble component (a "bare" component) with an enteric coating-film which is soluble at pH 5.0 to 7.0, preferably at pH 5.5 to 6.5. In brief, the slow-release portion may be prepared from a "bare" component by covering it with such a film.

The excipients which may be employed in preparing the slow-release portions used in this invention may be used in an amount of up to 75%, preferably 15 to 50%, by weight based upon the total amount of "bare" component of the slow-release portion and depending upon the desired dosage form. A preparation at the lowest limit, means, for example, microcapsules; since CCL per se is well absorbed in the upper part of the small intestine it may be formulated into microcapsules covered with a film soluble at pH 5.0 to 7.0, preferably at pH 5.5 to 6.5, by known methods for microcapsulation, and, additionally, in order to increase the absorption rate it may be formulated first into powder with suitable additives and then into microcapsules by the aforementioned methods.

The excipients, which are usually employed at the ratio mentioned above, include e.g., additives for preparing powder, fine granules, granules and beads, such as, e.g., sugar, sugar alcohols, starches and celluloses. Sugars such as dextrose, sucrose and lactose; sugar alcohols such as D-mannitol, sorbitol and inositol; starches such as wheat starch, corn starch and potato starch; and celluloses (high molecular weight compounds) such as crystalline cellulose, carboxymethylcellulose (hereinafter referred to as CMC), carboxymethylcellulose calcium (CMC-Ca), hydroxypropylcellulose (HPC), lower-substituted hydroxypropylcellulose (L-HPC) and hydroxypropylmethylcellulose (HPMC) are suitable examples. One or more additives selected from the above may be added to the formulation at the aforesaid ratio, preferably, and taking into consideration the desired dissolution rate and the stability of the formulation, it is appropriate to use at least one additive selected from D-mannitol, corn starch, crystalline cellulose and lower-substituted hydroxypropylcellulose as an excipient.

Additionally, suitable binders, if desired, with, e.g., suitable lubricants, disintegrators or excipients may be employed in preparing powder, fine granules, granules and beads in a conventional manner. The type of binder and the amount to be added must be carefully determined since they greatly influence the dissolution rate of CCL. Binders which may be employed include e.g., methylcellulose (MC), HPC, L-HPC, HPMC, dextrin, gelatin and starch.

It is generally difficult to fix the amount and type of binders to be added because they vary with the dosage form, the density of the formulation, and the amount and kind of excipients used; it is preferred to prepare the "bare" component (uncoated slow-release portion) so as rapidly to release the active ingredient. For example, when D-mannitol and MC are respectively employed as excipient and binder in preparing "bare" granules by a wet granulation method, they are usually added in amounts of 18 to 23 wt% and 0.9 to 1.3 wt%, respectively.

The "bare" component prepared in such a manner may be covered with an enteric coating film soluble at pH 5.0 to 7.0, preferably pH 5.5 to 6.5, to give the slow-release component.

Generally in this invention, enteric coating substances usually employed in preparing enteric formulations are applied onto the "bare" component in a conventional manner; more particularly, it is appropriate to prepare the enteric coating film as strongly to resist acid but rapidly to dissolve at pH 5.0 to 7.0, preferably pH 5.5 to 6.5, in order to prolong the action of CCL in the formulations of this invention, since the absorption site for CCL is limited in its location.

The enteric coating substances which may be employed include, e.g., phthalic acid celluloseacetate, hydroxypropylmethylcellulosephthalate (HPMCP), polyvinyl alcohol phthalate, carboxymethylethylcellulose, a copolymer of styrene and maleic acid, and a copolymer of methacrylic acid and methyl methacrylate, and, if desired, they may be employed with suitable plasticizers and/or extending agents.

The thus-prepared slow-release components are combined with the rapid-release components in the aforementioned ratios to give the long-acting formulations of CCL of this invention. "Rapid-release component" in this invention means a rapid-release formulation per se or a portion of the long-acting formulation which is well disintegrated and dissolved in the stomach. The component may be used in any dosage form; for example, powder, fine granules, granules or tablets, which may be prepared by admixing CCL with suitable excipients, if desired, along with e.g., lubricants; or native CCL (not formulated) can also be used. Additionally, the aforementioned "bare" component, which means an uncoated portion of the slow-release component, can also be used as the rapid-release component. More particularly, the rapid-release component may be a portion of multi-layer granules or beads prepared by spray-coating a rapid-release component onto a slow-release component prepared as described above. In preparing such multi-layer formulations, the thickness of the layer of the rapid-release portion needs to be estimated so that the ratio of the rapid release portion to the slow-release portion (by potency of CCL) is at the desired value.

Long-acting formulations of CCL in accordance with this invention were tested on some microorganisms or strains (Fig. 4; Experiment 4) in the same manner as described with reference to Fig. 3 (Experiment 3). As a consequence, it was confined that the 4:6 formulation (by potency of CCL), which was now expected to be the most satisfactory, gave satisfactory results for all the aforementioned items i. to iii. on all strains of microorganisms examined. In other words, at the blood-levels produced by the 4:6 formulation of CCL, the test microorganisms are sterilized rapidly and strongly and the effect is maintained over a long period of time.

These facts suggest that the formulations of this invention give sufficient clinical efficacy at less frequent administration, i.e., twice a day, than ordinary formulations do at the same daily dose without increasing the daily dose. In fact, it was recognized that the possible clinical efficacy of the formulations of this invention could be comparable to or better than that of ordinary formulations applied three times a day.

The long-acting formulations of CCL of this invention increase their antimicrobial activity through the long-acting effect, and are beneficial in being handy and simple in administration as mentioned above. Thus, the decrease of clinical efficacy resulting from failure of administration which has often occurred with ordinary formulations, can be avoided by the formulations of the present invention.

As mentioned above, the long-acting formulations of the present invention act continuously and directly on microorganisms greatly to increase the clinical efficacy of CCL along with preventing the failure of administration problem.

Experiment 1

Comparison between CEX and CCL for the blood levels (Fig. 1).

(1) Drugs:

250 mg Capsules of Keflex® (Shionogi & Co., LTD-Lilly) as CEX and 250 mg capsules of Kefral® (Shionogi & Co., LTD-Lilly) as CCL were employed.

(2) Subjects (12 volunteers; cross-over testing):

The subjects, 12 healthy and adult men aged 21 to 39 years with 1.58 to 1.83 $m^2$ body surface area.

(3) Administration:

Each subject after fasting (no meal after supper on the previous day) was given orally the capsules with 100 ml of warm water, and fasting was continued for 2 hours except for a prefixed amount of water. CEX and CCL were given to the 12 subjects in a cross-over method, and any drugs other than CEX or CCL were not applied for one week.

(4) Measurement:

The blood was collected 8 times, just before administration and after 0.5, 0.75, 1, 1.5, 2, 3 and 6 hours. The plasma was centrifugally separated with cooling immediately after each collection, rapidly frozen and then preserved at −20°C until the time of measurement.

The measurements were made within a week after collection. After the frozen samples were allowed to melt at 4°C, a series of ten-fold dilutions was prepared with 0.1 M of phosphate buffer (pH 6.0), in which the blood level was measured by a band culture method using Micrococcus luteus ATCC 9341 as a standard. Antibiotic Medium No. 8 (Difco; hereinafter referred to as ABM 8) was employed as medium.

(5) Result:

CEX and CCL resembled each other in the trends of their respective blood level curves, but it was confirmed that CCL showed lower maximum blood levels and was excreted faster than CEX.

Experiment 2

Time and concentration required for 99% sterilization (Fig. 2):

(1) Test organisms and their susceptibilities

TABLE 1

| Test microorganism | MICs (μg/ml) | |
|---|---|---|
| | CCL | CEX |
| *Escherichia coli* NIH JC-2 | 3.13 | 6.25 |
| *Escherichia coli* No. 29 | 1.56 | 3.13 |

Note: Minimum Inhibitory Concentrations (MICs) were assayed according to the dilution method disclosed in Chemotherapy 29(1), 76—79 (1981).

(2) Medium:
Heart infusion broth (Nissui Pharmaceutical Co.)

(3) Test method:
The test microorganisms in the logarithmic growth phase (about $10^6$ CFU/ml) were spread into the aforementioned broth placed in tubes, to which CCL or CEX was applied at prefixed serial concentrations. Changes in the number of viable microorganisms were monitored in order to measure the time required for 99% sterilization of the inoculum size in each tube.

(4) Result:
In order to diminish 99% of the inoculum size, CEX needed to be applied at a concentration of one or more MICs, but CCL was effective at a lower concentration, i.e. even at 1/2 MIC. It was also recognized that the time required for 99% sterilization by CCL was much shorter at any MIC of 1.2 to about 64 than for CEX.

TABLE 2
Time for 99%-sterilization

a. *E. coli* NIH JC-2

| | 4 MIC | 2 MIC | 1 MIC | 1/2 MIC | 1/4 MIC |
|---|---|---|---|---|---|
| CCL | 0° 35′ | 0° 35′ | 1° 00′ | 1° 30′ | — |
| CEX | 0° 55′ | 1° 00′ | 2° 00′ | — | — |

b. *E. coli* No. 29

| | 4 MIC | 2 MIC | 1 MIC | 1/2 MIC | 1/4 MIC |
|---|---|---|---|---|---|
| CCL | 1° 00′ | 1° 05′ | 2° 00′ | 2° 10′ | — |
| CEX | 2° 10′ | 2° 20′ | — | — | — |

Experiment 3
Growth-curve on the simulation of single-dose blood-level (Fig. 3):

(1) Test microorganism:
*Staphylococcus aureus* No. 1037

(2) Susceptibility of the test microorganism (MIC value):
1.56 µg/ml: The value was determined by the above-identified method, where Muller-Hinton agar (Difco) as the medium was employed—Method 1.
3.13 µg/ml: The value was determined by use of another medium, i.e., Antibiotic medium No. 3 (Difco; hereinafter referred to as ABM 3) broth in which the MIC was measured 18 to 20 hours after the inoculation ($10^5$ CFU/ml)—Method 2.

(3) Test method:
Time dependent changes in CCL blood levels were simulated in ABM 3 broth by means of computer simulation based on the CCL blood-level-curves which were obtained in Experiment 5. The number of viable cells after inoculation ($10^5$ CFU/ml) was monitored from time to time in the broth containing CCL as concentrations simulated by computer.
Such experiments were carried out on the CCL blood levels (at a dose of CCL 375 mg by potency) which were obtained from 11 kinds of 0:10 to 10:0 ratio formulations (rapid:slow potency of CCL). Results are illustrated in Fig. 3.

(4) Result:
In growth curves on the 11 kinds of formulations, the rate of decrease in number of viable cells (the slopes of the curves in the region A.) and the rate of increase after their revegetation (the slopes of the curves in the region B.) were very close; but there were significant differences in each datum for the 11 formulations, i.e., the time when the viable cells began to decrease and the time and the number of viable cells when they began to increase. Above all, the formulations of ratio 10:0 to 3:7 (rapid:slow) began to decrease in viable cell numbers earlier than the others; in the formulations of ratio 6:4 to 1:9 the revival of the organism was much delayed and the number of viable cells at that time was lower. In particular, it was found that the 4:6 formulation satisfied all the desired conditions, and the start of revival was most delayed, at which time the number of viable cells was the least.

Experiment 4

Growth-curve on simulation of a single dose (Fig. 4):

The same procedures as in Experiment 3. were applied to the following strains. The following MIC values were also determined according to the same method as in Experiment 3.

TABLE 3

| Test microorganism | | MICs (μg/ml) | |
|---|---|---|---|
| | | $10^5$ CFU/ml*1 | $10^5$ CFU/ml*2 |
| Gram (+) | *Staphylococcus aureus* No. 1021 | 1.56 | 3.13 |
| | *Staphylococcus aureus* No. 6 | 3.13 | 6.25 |
| Gram (−) | *Escherichia coli* ES-80 | 1.56 | 3.13 |
| | *Escherichia coli* ES-68 | 1.56 | 3.13 |
| | *Escherichia coli* ES-69 | 3.13 | 3.13 |

Note: The values with *1 or *2 were determined by method 1 or 2 disclosed in Experiment 3, respectively.

(Result):

In all of the strains of microorganisms, the results had a tendency similar to those in Experiment 3. On the basis of the facts, it was confirmed that the 4:6 formulation was preferred.

Experiment 5

Blood level of CCL with rapid-release or slow-release formulation:

(1) Dosage form, Dose:

491 mg of the rapid-release granules (375 mg as CCL) prepared in Example 2. or 713 mg of the slow-release granules (375 mg as CCL) were administered to the following 4 subjects.

(2) Subjects (4 adult men) were:

4 healthy adult men, aged 26 to 46 years, with 53 to 65 kg body-weight.

(3) Manner of administration:

The rapid-release granules identified above were administered to the 4 subjects 30 minutes after regular meals, and the slow-release granules to the same four subjects after a period of one week in duration during which no drug was given.

(4) Measurement of CCL in plasma:

Blood samples were collected from the 4 treated subjects at prefixed times (total 7 times for each subject) as shown in Table 4, and CCL concentrations in the plasma were measured by High Performance Liquid Chromatography (HPLC).

(5) Result:

TABLE 4
CCL concentrations in plasma (μg/ml)

(Rapid-release granules)

| Subject | Time (hr) | | | | | | |
|---|---|---|---|---|---|---|---|
| | 0.5 | 1.0 | 2.0 | 3.0 | 4.0 | 6.0 | 8.0 |
| A | 6.51 | 4.48 | 2.36 | 1.38 | 1.04 | 0 | 0 |
| B | 3.30 | 3.50 | 3.83 | 4.50 | 2.04 | 0.30 | 0 |
| C | 5.70 | 4.56 | 3.85 | 2.31 | 0.95 | 0.20 | 0 |
| D | 2.07 | 4.37 | 3.88 | 3.53 | 3.18 | 0.40 | 0 |
| Average | 4.40 ±1.03 | 4.23 ±0.25 | 3.48 ±0.25 | 2.93 ±0.68 | 1.80 ±0.52 | 0.23 ±0.09 | 0 |

(Slow-release granules)

| Subject | Time (hr) | | | | | | |
|---|---|---|---|---|---|---|---|
| | 1.0 | 2.0 | 3.0 | 4.0 | 6.0 | 8.0 | 10.0 |
| A | 0 | 1.82 | 2.53 | 1.38 | 1.92 | 0.34 | 0 |
| B | 0.23 | 0.55 | 2.08 | 3.11 | 2.15 | 0.33 | 0 |
| C | 0 | 1.59 | 3.01 | 2.70 | 1.73 | 0.38 | 0 |
| D | 0 | 0 | 0.22 | 1.56 | 4.39 | 1.01 | 0 |
| Average | 0.06 ±0.06 | 0.99 ±0.43 | 1.96 ±0.61 | 2.19 ±0.42 | 2.55 ±0.62 | 0.52 ±0.33 | 0 |

Experiment 6

Comparison of clinical efficacy by double-blind tests between the present long-acting formulations and ordinary formulations of CCL:

(1) Subjects to be treated:

Patients, aged 15 years or more, with dental infections which would be expected to respond clinically to treatment with the ordinary formulations at a 750 mg daily dose of CCL (3 divided doses) were selected, except for the following types of patients:

a. patients suffering from a functional disorder in the kidney or liver, or other serious disease,

b. patients treated with an antibacterial or antifungal agent or a steroid hormone recently before the test,

c. patients having a history of allergy to cephalosporin- or penicillin-type agents, and

d. patients in pregnancy or nursing mothers.

(2) Formulation:

The long-acting formulations of CCL were prepared as granules, enclosed in a package, containing a total of 375 mg of CCL at a ratio of 4:6 (rapid:slow) by potency. The ordinary formulations were prepared as capsules, each of which contained 250 mg of CCL by potency as an unprocessed formulation. Furthermore, two kinds of placebos indentical in external appearance to the packages of granules and the capsules were prepared.

(3) Administration:

According to the schedule shown in the following Table 5, both a package of granules and a capsule were orally administered 3 times a day after every meal. In this schedule, each daily dose of CCL is 750 mg by potency in both the long-acting formulation group (Group L) and the ordinary formulation group (Group R).

The administration was generally continued for 5 days.

TABLE 5

| | | morning | noon | evening | a daily dose |
|---|---|---|---|---|---|
| Group L | granules | CCL (375 mg) | placebo | CCL (375 mg) | CCL 750 mg |
| | capsule | placebo | placebo | placebo | by potency |
| Group R | granules | placebo | placebo | placebo | CCL 750 mg |
| | capsule | CCL (250 mg) | CCL (250 mg) | CCL (250 mg) | by potency |

Table 5 indicates that in Group L, CCL was administered twice a day, i.e., in the morning and the evening, in a form of a long-acting formulation (daily dose: 750 mg of CCL by potency), and in Group R, 3 times a day, i.e. in the morning, at noon and in the evening, in the form of an ordinary formulation (daily dose: 750 mg of CCL by potency).

In addition, all sets of formulations for both groups were prepared to have the same form of external appearance so that it was impossible to distinguish one from the other.

(4) Result:

Efficacy was evaluated according to "The criteria for evaluation of antimicrobial agents in oral surgery" edited by Japanese Society of Oral Therapeutics and Pharmacology.

The clinical efficacy in Group L was as high as in Group R.

TABLE 6

| | Clinical efficacy on the 5th day | | | |
|---|---|---|---|---|
| | Excellent | Well | None | Total |
| Group L | 69 | 16 | 4 | 89 |
| | 95.5% | | | |
| Group R | 69 | 18 | 3 | 90 |
| | 96.7% | | | |
| Total | 137 | 35 | 7 | 179 |
| | 96.1% | | | |

In another aspect, the present invention, of course, provides a method of preparing a long-acting formulation of cefaclor which comprises formulating together to provide a single formulation, a non-enteric coated component of cefaclor to serve as a rapid-release component and an enteric coated component of cefaclor to serve as a slow-release component at a ratio of about 4:6 respectively based on potency of cefaclor wherein said non-enteric coated component contains at least one additive selected from lactose, sucrose, D-mannitol, corn starch, wheat starch and low-substituted hydroxypropylcellulose in an amount of up to 75% weight based on the whole non-enteric coated component and said enteric-coated component contains at least one additive selected from lactose, sucrose, D-mannitol, corn starch, wheat starch and low-substituted hydroxypropylcellulose in an amount of up to 75% weight based on the whole enteric-coated component and an enteric-coating which is a film soluble in the pH range 5.0 to 7.0.

In the following Examples, given further to illustrate and describe the invention, Examples 1 to 7 relate to rapid-release components, Examples 8 to 11 and 13 relate to slow-release components, and Examples 12 and 14 to 17 relate to overall formulations in accordance with the invention.

Example 1

| Composition | (% w/w) |
|---|---|
| cefaclor | 76.3 |
| lactose | 11.9 |
| corn starch | 7.8 |
| potato starch | 1.5 |
| Moisture | 2.5 |
| Total | 100 |

To a mixture of 1912 g of cefaclor, 298 of lactose and 222 g of corn starch was added 730 g of a 5% paste of potato starch. The mixture was kneaded and formulated into granules with a rotary wet granulator, which were then dried at 50°C for one hour. The dried granules were rounded off by a speed-mill and sifted out to give bare granules A which pass through a 14 mesh sieve but not through a 24 mesh one.

In this invention, the content of cefaclor in compositions is shown as an amount by weight derived from the CCL potency of the overall composition, e.g., a weight by potency based on the total of e.g., dried granules or other composition form. This rule is applied in the process for preparing the formulations, too.

Example 2

| Composition | (% w/w) |
|---|---|
| cefaclor | 76.3 |
| corn starch | 12.6 |
| L-HPC | 6.4 |
| HPC | 2.1 |
| Moisture | 2.6 |
| Total | 100 |

To a mixture of 1912 g of cefaclor, 356 g of corn starch and 162 g of L-HPC was added 960 g of 5.5% HPC aqueous solution. The mixture was kneaded and then formulated into bare granules B in the same manner as in Example 1.

Example 3

| Composition | (% w/w) |
|---|---|
| cefaclor | 76.3 |
| D-mannitol | 6.8 |
| corn starch | 6.7 |
| L-HPC | 6.4 |
| methylcellulose 25 mPas | 1.3 |
| Moisture | 2.5 |
| Total | 100 |

To a mixture of 1912 g of cefaclor, 170 g of D-mannitol, 191 g of corn starch and 162 g of L-HPC was added 940 g of 3.3% aqueous methylcellulose (25 mPas). The mixture was kneaded and formulated into bare granules C in the same manner as in Example 1.

The respective bare granules A to C prepared in Examples 1 to 3 contain 763 mg of CCL by potency per gram.

Example 4

| Composition | (% w/w) |
|---|---|
| cefaclor | 46.6 |
| lactose | 29.5 |
| corn starch | 19.9 |
| potato starch | 1.6 |
| Moisture | 2.4 |
| Total | 100 |

To a mixture of 1275 g of cefaclor, 808 g of lactose and 618 g of corn starch was added 810 g of 5% potato starch paste, the mixture kneaded and formulated into bare granules D in the same manner as in Example 1.

Example 5

| Composition | (% w/w) |
|---|---|
| cefaclor | 46.6 |
| corn starch | 45.3 |
| L-HPC | 3.9 |
| HPC | 1.9 |
| Moisture | 2.3 |
| Total | 100 |

To a mixture of 1125 g of cefaclor, 1243 g of corn starch and 94 g of L-HPC was added 560 g of 8% aqueous HPC. The mixture was kneaded and formulated into bare granules E in the same manner as in Example 1.

Example 6

| Composition | (% w/w) |
|---|---|
| cefaclor | 46.6 |
| D-mannitol | 23.6 |
| corn starch | 22.5 |
| L-HPC | 3.9 |
| methylcellulose 25 mPas | 1.1 |
| Moisture | 2.3 |
| Total | 100 |

To a mixture of 1125 g of cefaclor, 570 g of D-mannitol, 616 g of corn starch and 94 g of L-HPC was added 560 g of 5% aqueous methylcellulose (25 mPas). The mixture was kneaded and formulated into bare granules F in the same manner as in Example 1. The bare granules D to F prepared in Examples 4 to 6 contain 466 mg of CCL by potency per gram.

Example 7

| Composition | (% w/w) |
|---|---|
| cefaclor | 63.4 |
| sucrose | 34.4 |
| Macrogol 6000 | 0.7 |
| HPC | 0.7 |
| Moisture | 0.8 |
| Total | 100 |

An aqueous solution (a mixture of 1220 g of 2% aqueous Macrogel and 1220 g of 2% aqueous HPC) was sprayed onto a mixture of 2250 g of cefaclor and 1220 g of sucrose and dried in a fluidized-bed granulator; the spraying and the drying were repeated to give spherical beads. The beads were sifted out to 32—60 mesh particles as bare beads G. The beads G contain 634 mg of CCL by potency per gram.

Example 8

| Composition | (% w/w) |
|---|---|
| HPMCP | 5.812 |
| white shellac | 0.646 |
| glycerin fatty acid ester | 2.153 |
| talc | 5.389 |
| ethanol | 52.3 |
| dichloromethane | 33.7 |
| Total | 100 |

A coating-solution of the above identified composition was prepared. Onto 1000 g of bare granules A prepared in Example 1 placed in a coating pan of 40 cm in diameter was sprayed 3570 g of the solution in a conventional manner to give slow-release granules A-1. The bare granules B, C, D, E and F of Examples 2 to 6 were treated in the same manner as shown above to give slow-release granules B-1, C-1, D-1, E-1 and F-1, respectively. The slow-release granules A-1 to C-1 contain 526 mg, and D-1 to F-1 321 mg of CCL by potency per gram, respectively.

Example 9

| Composition | (% w/w) |
|---|---|
| Eudragit® L100 | 5.812 |
| white shellac | 0.646 |
| glycerin fatty acid ester | 2.153 |
| talc | 5.389 |
| ethanol | 86.0 |
| Total | 100 |

The above-identified coating-solution was prepared. In the same manner as shown in Example 9, 3570 g of the above-identified coating solution was sprayed onto bare granules A, B, C, D, E and F prepared in Examples 1 to 6 to give slow-release granules A-2, B-2, C-2, D-2, E-2 and F-2, respectively.

The slow-release granules A-2 to C-2 contain 526 mg and D-2 to F-2 321 mg of cefaclor by potency per gram, respectively.

Example 10

| Composition | (% w/w) |
|---|---|
| Eudragit® S100 | 5.812 |
| white shellac | 0.646 |
| glycerin fatty acid ester | 2.153 |
| talc | 5.389 |
| ethanol | 86.0 |
| Total | 100 |

The above-identified coating-solution was prepared.

In the same manner as shown in Example 8, 3570 g of the solution was sprayed onto bare granules A, B, C, D, E and F prepared in Examples 1 to 6 to give slow-release granules A-3, B-3, C-3, D-3, E-3 and F-3, respectively.

The slow-release granules A-3 to C-3 contain 526 mg and D-3 to F-3 321 mg of cefaclor by potency per gram, respectively.

Example 11

| Composition | (% w/w) |
|---|---|
| Eudragit® L30D | 47.2 |
| Macrogol 6000 | 1.4 |
| talc | 4.2 |
| purified water | 47.2 |
| Total | 100 |

The above-identified coating-solution was prepared. 1000 g lots of bare granules A, B, C, D, E and F prepared in Examples 1 to 6 were spray-coated with 2400 g of the solution by a fluidized-bed coating machine to give slow-release granules A-4, B-4, C-4, D-4, E-4 and F-4, respectively.

The slow-release granules A-4 to C-4 contain 526 mg and D-4 to F-4 321 mg of cefaclor by potency per gram, respectively.

Example 12

| Composition | (% w/w) |
|---|---|
| cefaclor | 10.0 |
| HPC | 6.7 |
| lactose | 3.3 |
| purified water | 80.0 |
| Total | 100 |

Onto 1000 g of slow-release granules A-2 prepared in Example was sprayed 3690 g of a suspension prepared according to the above prescription using in a conventional manner a fluidized-bed coating machine to provide rapid-release layers giving multi-layer granules I.

The ratio of CCL in the rapid-release portion of the multi-layer granules I to that in the slow-release portion was 4:6 by potency, with the granules I containing 516 mg of CCL by potency per gram.

Example 13

In a conventional manner using a fluidized-bed coating machine, 3970 g of the coating-solution prepared in Example 9 was sprayed onto 1000 g of bare beads G prepared in Example 7 to give slow-release beads J. The beads J contain 423 mg of CCL by potency per gram.

Example 14

Both the rapid-release granules and the slow-release granules as shown in the following were packed into strip-packages (hereinafter referred to as SP) to give blended formulations. The ratio of CCL in the rapid-release portion to that in the slow-release one was 4:6 by potency, with each package containing 375 mg of CCL by potency. The following shows the amounts of the corresponding granules packed in the various packages.

1) 196 mg of granules B and 428 mg of granules A-1
2) 322 mg of granules D and 428 mg of granules B-3
3) 322 mg of granules F and 428 mg of granules C-2
4) 196 mg of granules C and 700 mg of granules D-4
5) 322 mg of granules E and 700 mg of granules F-2

Thus, five kinds of blended granules (SP) were prepared.

Example 15

Both the following rapid-release and slow-release formulations were filled into a hard-type capsule to give six different kinds of capsules of long-acting CCL.

The ratio of CCL in the rapid-release portions to that in the slow-release portions was 3.5:6.5 by potency, with 187.5 mg of CCL by potency contained per capsule.

The contents below are shown as the weight of granules or beads loaded into a capsule.

1) 86 mg of the granules A and 232 mg of the granules A-3
2) 86 mg of the granules C and 232 mg of the granules B-1
3) 141 mg of the granules E and 232 mg of the granules C-2
4) 141 mg of the granules D and 232 mg of the granules C-4
5) 103 mg of the beads G and 288 mg of the beads J

Example 16

According to the procedures shown in Example 15, capsule-formulations of long-acting CCL, which contain 187.5 mg of CCL by potency per capsule, were prepared. The ratio of CCL in the rapid-release portions to that in the slow-release portions was 4:6 by potency.

1) 161 mg of the granules F and 214 mg of the granules B-3
2) 98 mg of the granules A and 350 mg of the granules D-1
3) 98 mg of the granules B and 214 mg of the granules A-4
4) 98 mg of the granules C and 214 mg of the granules C-2

Thus, four different kinds of capsules were prepared.

Example 17

According to the procedures shown in Example 15, capsules of long-acting CCL formulations, containing 187.5 mg of CCL by potency per capsule, were prepared. The ratio of CCL in the rapid-release portions to that in the slow-release portions was 4.5:5.5 by potency.

1) 111 mg of the granules B and 321 mg of the granules E-1
2) 111 mg of the granules C and 321 mg of the granules F-2
3) 181 mg of the granules E and 196 mg of the granules A-3
4) 181 mg of the granules D and 196 mg of the granules C-4

Thus, four different kinds of capsules were prepared.

## Claims

1. A long-acting formulation of cefaclor, comprising a mixture of a non-enteric coated component of cefaclor to serve as a rapid-release component with an enteric coated component of cefeclor to serve as a slow-release component at a ratio of about 4:6 respectively based on potency of cefaclor wherein said non-enteric coated component contains at least one additive selected from lactose, sucrose, D-mannitol, corn starch, wheat starch and low-substituted hydroxypropylcellulose in an amount of up to 75% weight based on the whole non-enteric coated component and said enteric coated component contains at least one additive selected from lactose, sucrose, D-mannitol, corn starch, wheat starch and low-substituted hydroxypropylcellulose in an amount of up to 75% weight based on the whole enteric-coated component, and an enteric-coating which is a film soluble in the pH range 5.0 to 7.0.

2. A long-acting formulation of cefaclor as claimed in claim 1 wherein said enteric coating film is soluble in a pH range of 5.5 to 6.5.

3. A long-acting formulation of cefaclor as claimed in any preceding claim wherein said enteric coating film is of methyl methacrylate-methacrylic acid copolymer.

4. A long-acting formulation of cefaclor as claimed in any preceding claim wherein the enteric-coated component is in the form of microcapsules, granules or beads.

5. A long acting formulation of cefaclor as claimed in any preceding claim which is in the form of capsules filled with said mixture.

6. A method of preparing a long-acting formulation of cefaclor which comprises formulating together to provide a single formulation, a non-enteric coated component of cefaclor to serve as a rapid-release component and an enteric coated component of cefaclor to serve as a slow-release component at a ratio of about 4:6 respectively based on potency of cefaclor wherein said non-enteric coated component contains at least one additive selected from lactose, sucrose, D-mannitol, corn starch, wheat starch and low-substituted hydroxypropylcellulose in an amount of up to 75% weight based on the whole non-enteric coated component and said enteric-coated component contains at least one additive selected from lactose, sucrose, D-mannitol, corn starch, wheat starch and low-substituted hydroxypropylcellulose in an amount of up to 75% weight based on the whole enteric-coated component and an enteric-coating which is a film soluble in the pH range 5.0 to 7.0.

7. A method as claimed in claim 6 further defined by the specific feature of any one of claims 2 to 5.

## Patentansprüche

1. Cefaclor-Formulierung mit Langzeitwirkung, enthaltend ein Gemisch einer magensaftlöslich beschichteten Cefaclor-Komponente, die als Komponente mit schneller Freisetzung dient, mit einer darmlöslich beschichteten Cefaclor-Komponente, die als Komponente mit langsamer Freisetzung dient, im Verhältnis von etwa 4:6, bezogen auf die Wirksamkeit des Cefaclors, wobei die magensaftlöslich beschichtete Komponente mindestens ein Additiv, ausgewählt aus der von Lactose, Sucrose, D-Mannit, Maisstärke, Weizenstärke und niedrigsubstituierter Hydroxypropylcellulose gebildeten Gruppe, in einer Menge bis zu 75 Gew.-%, bezogen auf das Gesamtgewicht der magensaftlöslich beschichteten Komponente, enthält, und die darmlöslich beschichtete Komponente mindestens ein Additiv, ausgewählt aus der von Lactose, Sucrose, D-Mannit, Maisstärke, Weizenstärke und niedrigsubstituierter Hydroxypropylcellulose gebildeten Gruppe, in einer Menge bis zu 75 Gew.-%, bezogen auf das Gesamtgewicht der darmlöslich beschichteten Komponente, und eine darmlösliche Beschichtung, die ein im pH-Bereich von 5,0 bis 7,0 löslicher Film ist, enthält.

2. Cefaclor-Formulierung mit Langzeitwirkung nach Anspruch 1, dadurch gekennzeichnet, daß der darmlösliche Beschichtungsfilm im pH-Bereich von 5,5 bis 6,5 löslich ist.

3. Cefaclor-Formulierung mit Langzeitwirkung nach einem der obigen Ansprüche, dadurch gekennzeichnet, daß der darmlösliche Beschichtungsfilm ein Methylmethacrylat-Methacrylsäure-Copolymer ist.

4. Cefaclor-Formulierung mit Langzeitwirkung nach einem jeden der obigen Ansprüche, dadurch gekennzeichnet, daß die darmlöslich beschichtete Komponente in Form von Microkapseln, Granulat oder Perlen vorliegt.

5. Cefaclor-Formulierung mit Langzeitwirkung nach einem jeden der obigen Ansprüche, dadurch gekennzeichnet, daß sie in Form von Kapseln vorliegt, die mit dem beanspruchten Gemisch gefüllt sind.

6. Verfahren zur Herstellung einer Cefaclor-Formulierung mit Langzeitwirkung bei dem zur Bildung einer einheitlichen Formulierung eine magensaftlöslich beschichtete Cefaclor-Komponente, die als Komponente mit schneller Freisetzung dient, und eine darmlöslich beschichtete Cefaclor-Komponente, die als Komponente mit langsamer Freisetzung dient, im Verhältnis von etwa 4:6, bezogen auf die Wirksamkeit des Cefaclors, zusammenformuliert werden, wobei die magensaftlöslich beschichtete Komponente mindestens ein Additiv, ausgewählt aus der von Lactose, Sucrose, D-Mannitol, Maisstärke, Weizenstärke und niedrigsubstituierter Hydroxypropylcellulose gebildeten Gruppe, in einer Menge bis zu 75 Gew.-%, bezogen auf das Gesamtgewicht der magensaftlöslich beschichteten Komponente, enthält, und die darmlöslich beschichtete Komponente mindestens ein Additiv, ausgewählt aus der von Lactose, Sucrose, D-Mannitol, Maisstärke, Weizenstärke und niedrigsubstituierter Hydroxypropylcellulose gebildeten Gruppe, in einer Menge bis zu 75 Gew.-%, bezogen auf das Gesamtgewicht der darmlöslich beschichteten Komponente, und eine darmlösliche Beschichtung, die ein im pH-Bereich von 5,0 bis 7,0 löslicher Film ist, enthält.

7. Verfahren nach Anspruch 6, weiter gekennzeichnet durch die Merkmale eines jeden der Ansprüche 2 bis 5.

**Revendications**

1. Formulation retard de céfaclor, comprenant un mélange d'un dérivé de céfaclor non-enrobé, destiné à servir de composant à libération rapide, et d'un dérivé de céfaclor enrobé gastro-résistant destiné à servir de composant à libération lente, selon une proportion, basée sur l'activité du céfaclor, d'environ 4:6, ledit dérivé non-enrobé contenant au moins un additif choisi dans le groupe comprenant le lactose, la saccharose, le D-mannitol, l'amidon de maïs, l'amidon de blé et de l'hydroxypropylcellulose à faible degré de substitution en une quantité allant jusqu'à 75% en poids par rapport à la totalité du composant non-enrobé, ledit composant enrobé contenant au moins un additif choisi dans le groupe comprenant le lactose, le saccharose, le D-mannitol, l'amidon de maïs, l'amidon de blé et de l'hydroxypropylcellulose à faible degré de substitution en une quantité allant jusqu'à 75% en poids par rapport à la totalité du composant enrobé gastro-résistant, et un enrobage gastro-résistant, qui est un film soluble à un pH compris entre 5,0 et 7,0.

2. Formulation retard de céfaclor selon la revendication 1, dans laquelle ledit film d'enrobage gastro-résistant est soluble à un pH compris entre 5,5 et 6,5.

3. Formulation retard de céfaclor selon l'une quelconque des revendications précédentes, dans laquelle ledit film d'enrobage gastro-résistant est constitué d'un copolymère de méthacrylate de méthyle et d'acide méthacrylique.

4. Formulation retard de céfaclor selon l'une quelconque des revendications précédentes, dans laquelle le composant enrobé gastro-résistant se présente sous la forme de microcapsules, de granules ou de perles.

5. Formulation retard de céfaclor selon l'une quelconque des revendications précédentes, qui se présente sous la forme de capsules remplies dudit mélange.

6. Procédé de préparation d'une formulation retard de céfaclor, qui consiste à formuler ensemble, pour donner une formulation unique, un dérivé de céfaclor non-enrobé, destiné à servir de composant à libération rapide, et un dérivé de céfaclor enrobé gastro-résistant destiné à servir de composant à libération lente, selon une proportion, basée sur l'activité du céfaclor, d'environ 4:6, ledit dérivé non-enrobé contenant au moins un additif choisi dans le groupe comprenant le lactose, le saccharose, le D-mannitol, l'amidon de maïs, l'amidon de blé et de l'hydroxypropylcellulose à faible degré de substitution en une quantité allant jusqu'à 75% en poids par rapport à la totalité du composant non-enrobé, ledit composant enrobé contenant au moins un additif choisi dans le groupe comprenant le lactose, le saccharose, le D-mannitol, l'amidon de maïs, l'amidon de blé et de l'hydroxypropylcellulose à faible degré de substitution en une quantité allant jusqu'à 75% en poids par rapport à la totalité du composant enrobé gastro-résistant, et un enrobage gastro-résistant, qui est un film soluble à un pH compris entre 5,0 et 7,0.

7. Procédé selon la revendication 6, défini en outre par les caractéristiques particulières de l'une quelconque des revendications 2 à 5.

FIG. 1.

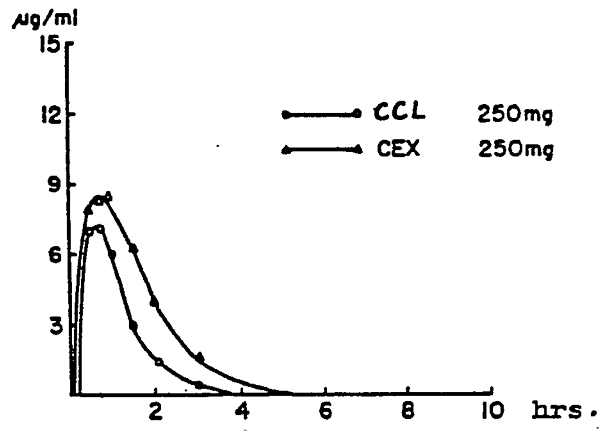
µg/ml
15
12
9
6
3
2
4
6
8
10
hrs.
CCL 250mg
CEX 250mg

FIG. 2.

A.

B.

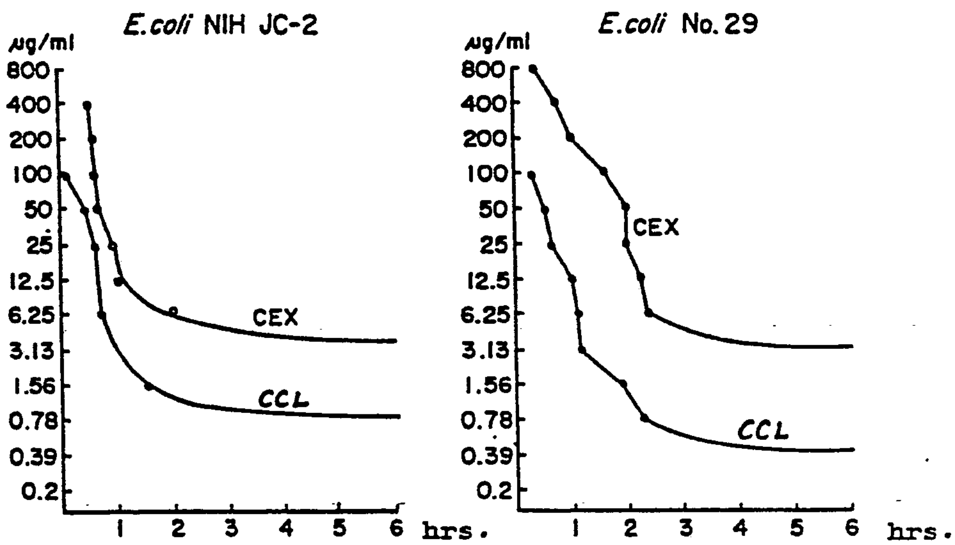
E.coli NIH JC-2
µg/ml
800
400
200
100
50
25
12.5
6.25
3.13
1.56
0.78
0.39
0.2
1
2
3
4
5
6
hrs.
CEX
CCL
E.coli No. 29
µg/ml
800
400
200
100
50
25
12.5
6.25
3.13
1.56
0.78
0.39
0.2
1
2
3
4
5
6
hrs.
CEX
CCL

S. aureus No.1037 : CCL 375mg compound granules

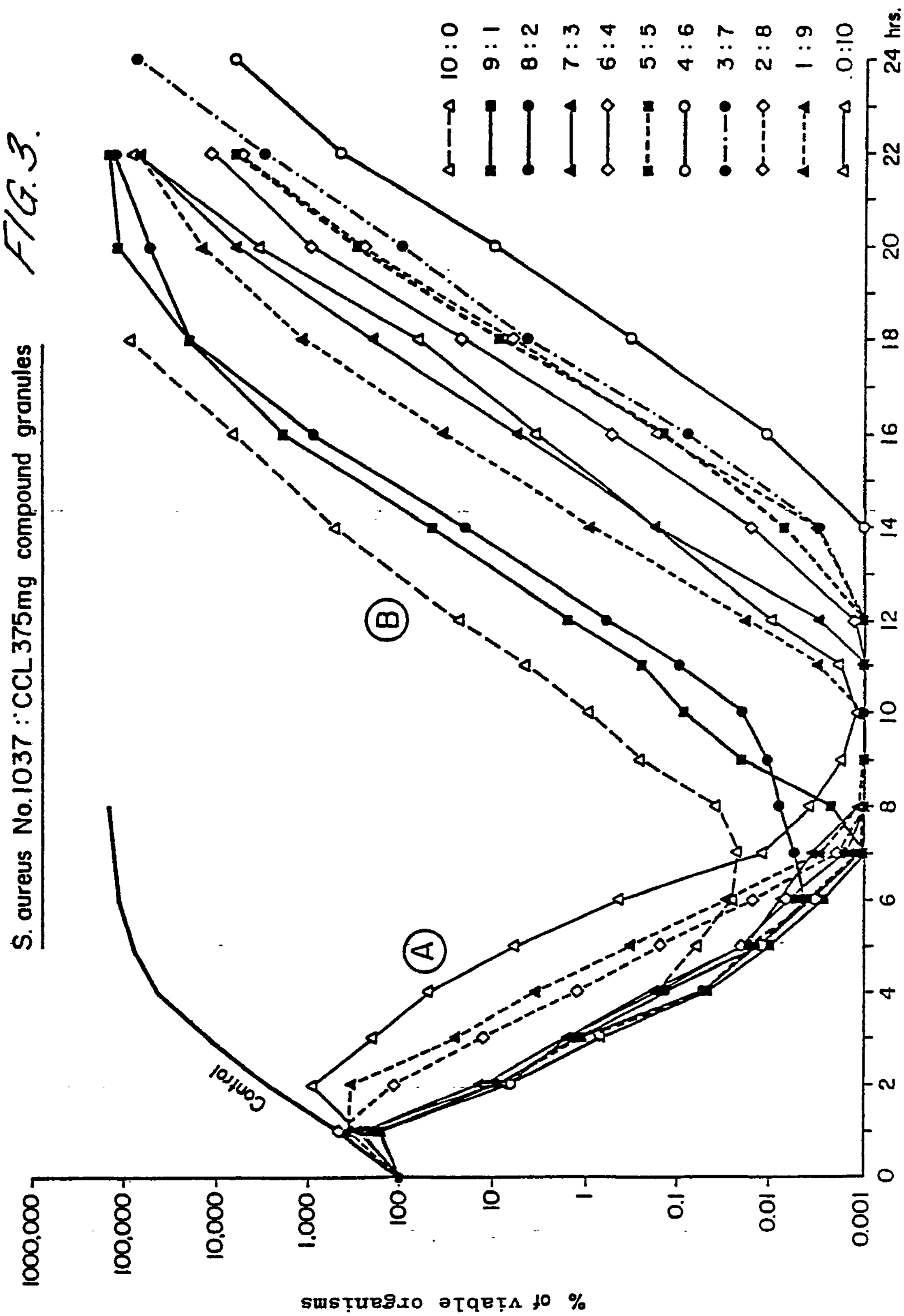


FIG. 3.

FIG. 4a.

S. aureus No.1021 : CCL 375mg compoud granules

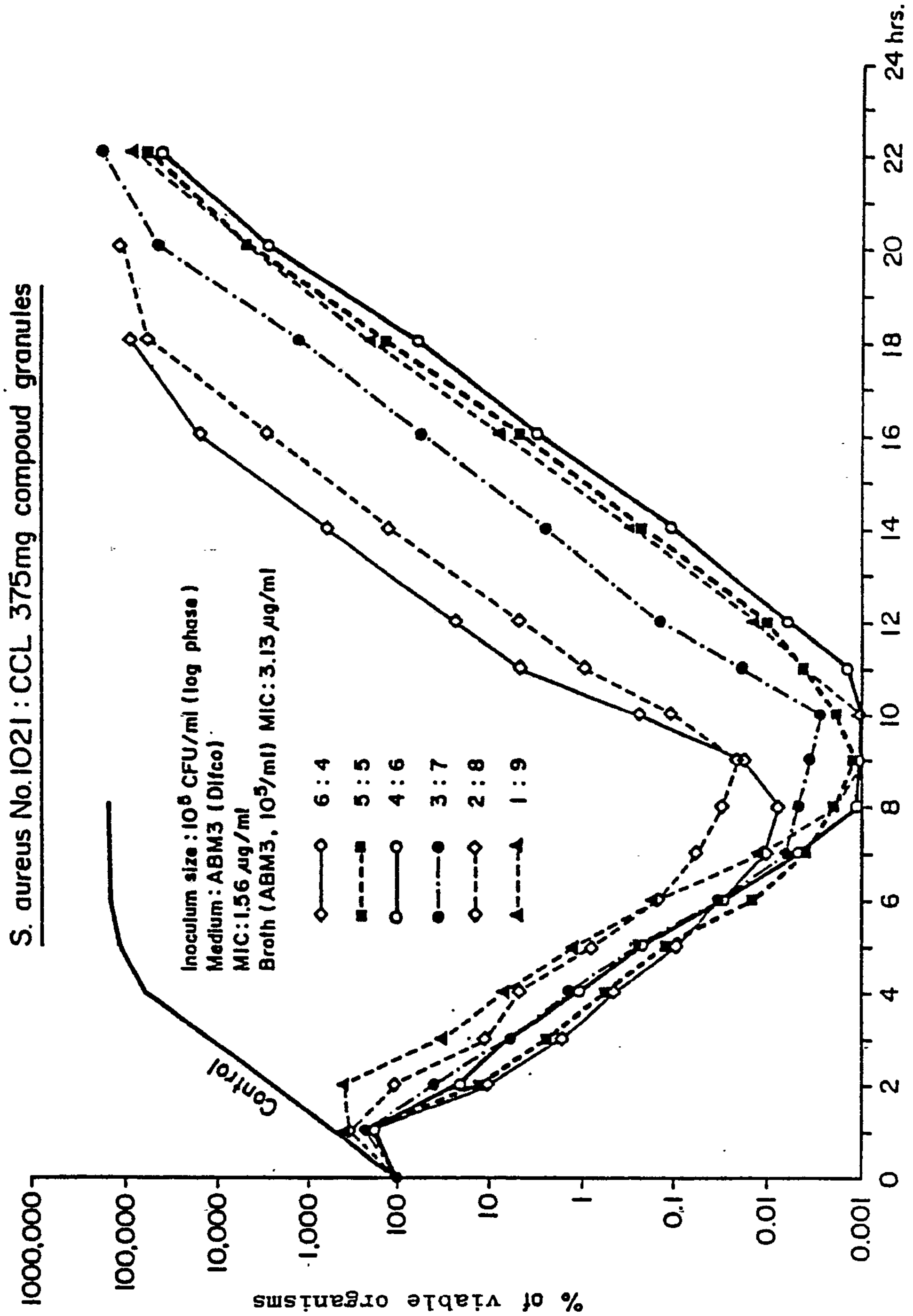

FIG. 4b.

S. aureus No.6 : CCL 375mg compound granules

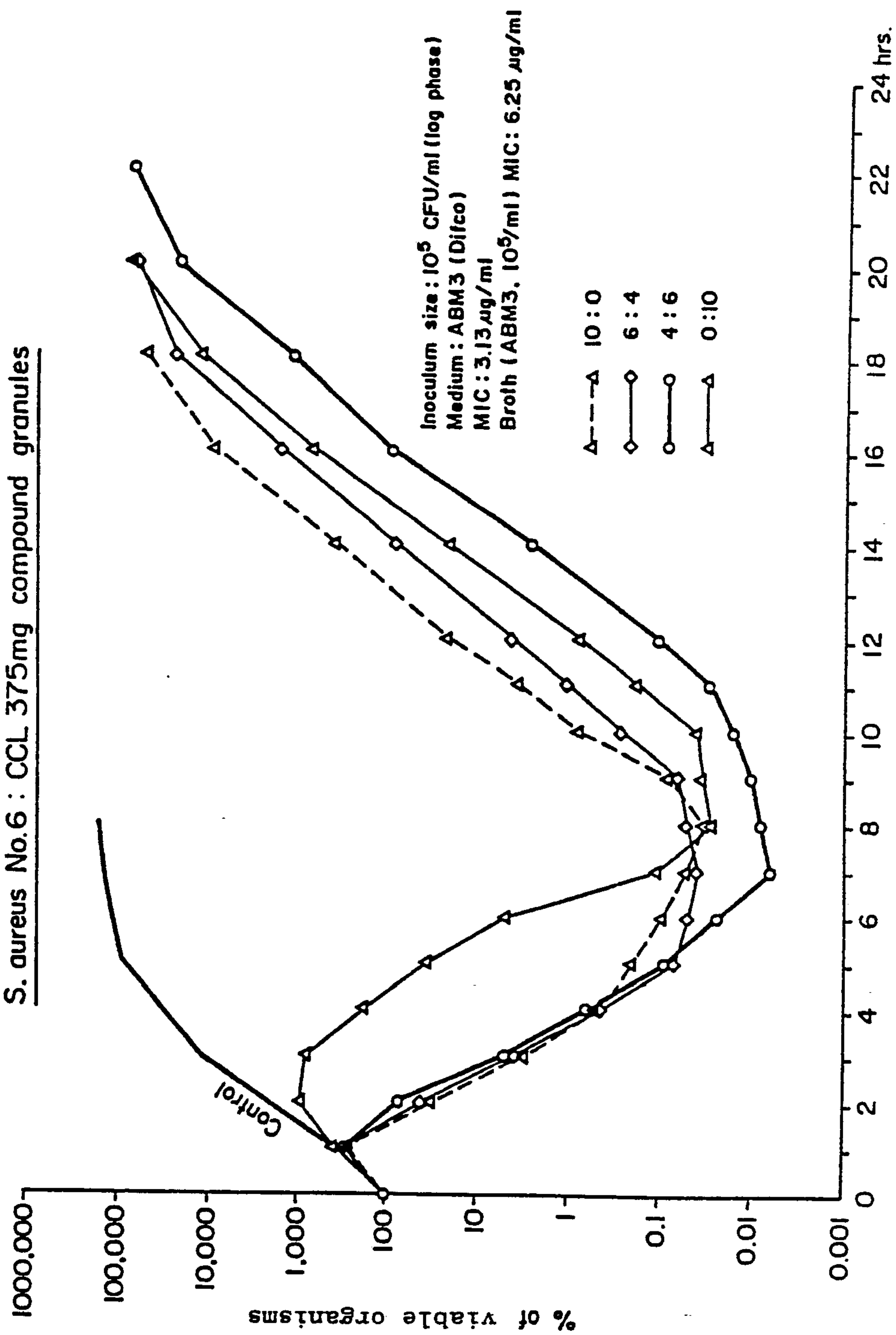

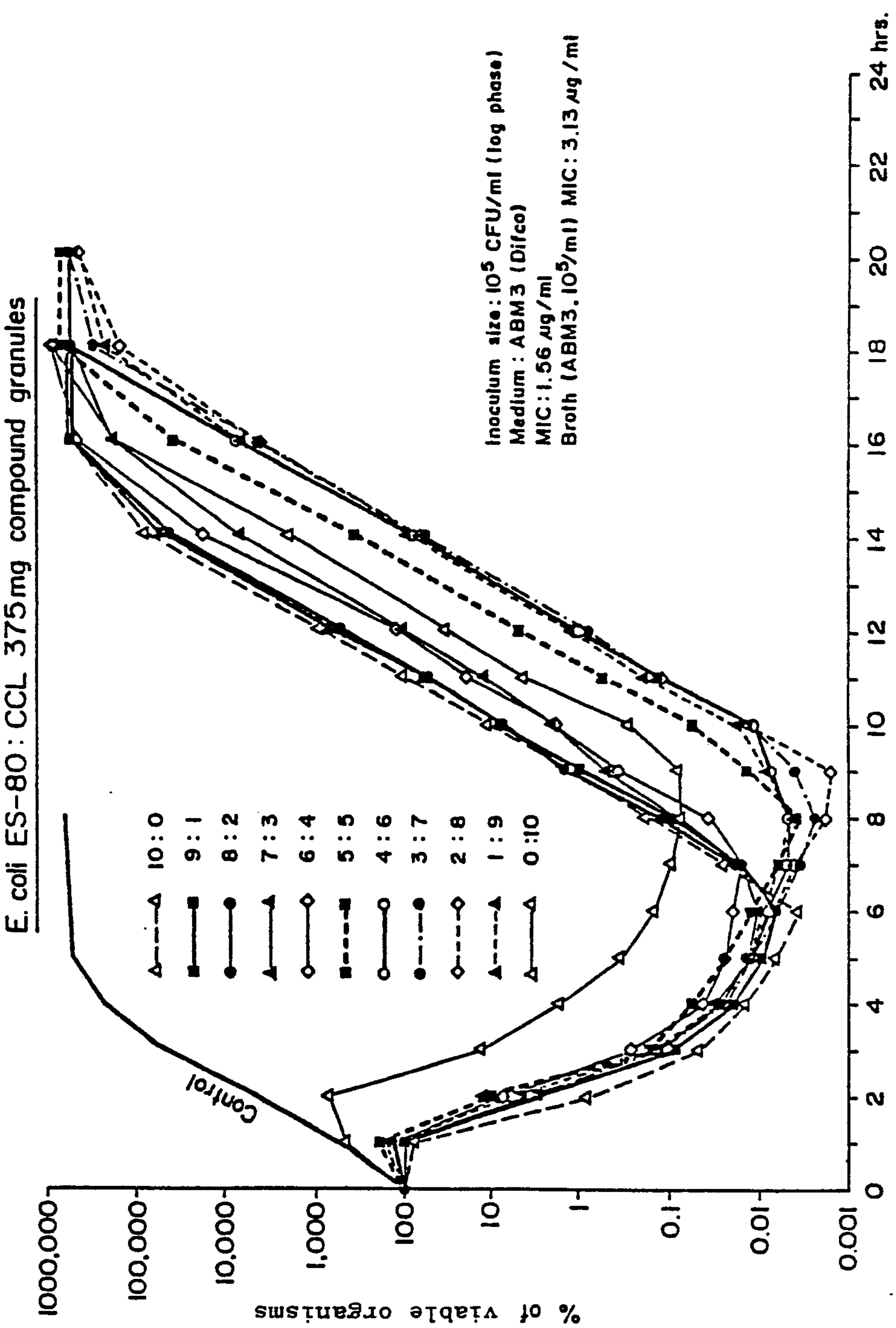
E. coli ES-80 : CCL 375mg compound granules
10 : 0
9 : 1
8 : 2
7 : 3
6 : 4
5 : 5
4 : 6
3 : 7
2 : 8
1 : 9
0 : 10
Control
Inoculum size: $10^5$ CFU/ml (log phase)
Medium: ABM3 (Difco)
MIC: 1.56 µg/ml
Broth (ABM3, $10^5$/ml) MIC: 3.13 µg/ml
% of viable organisms
1000,000
100,000
10,000
1,000
100
10
1
0.1
0.01
0.001
0
2
4
6
8
10
12
14
16
18
20
22
24 hrs.

FIG. 4C.

FIG. 4d.

E. coli ES-68 : CCL 375mg compound granules

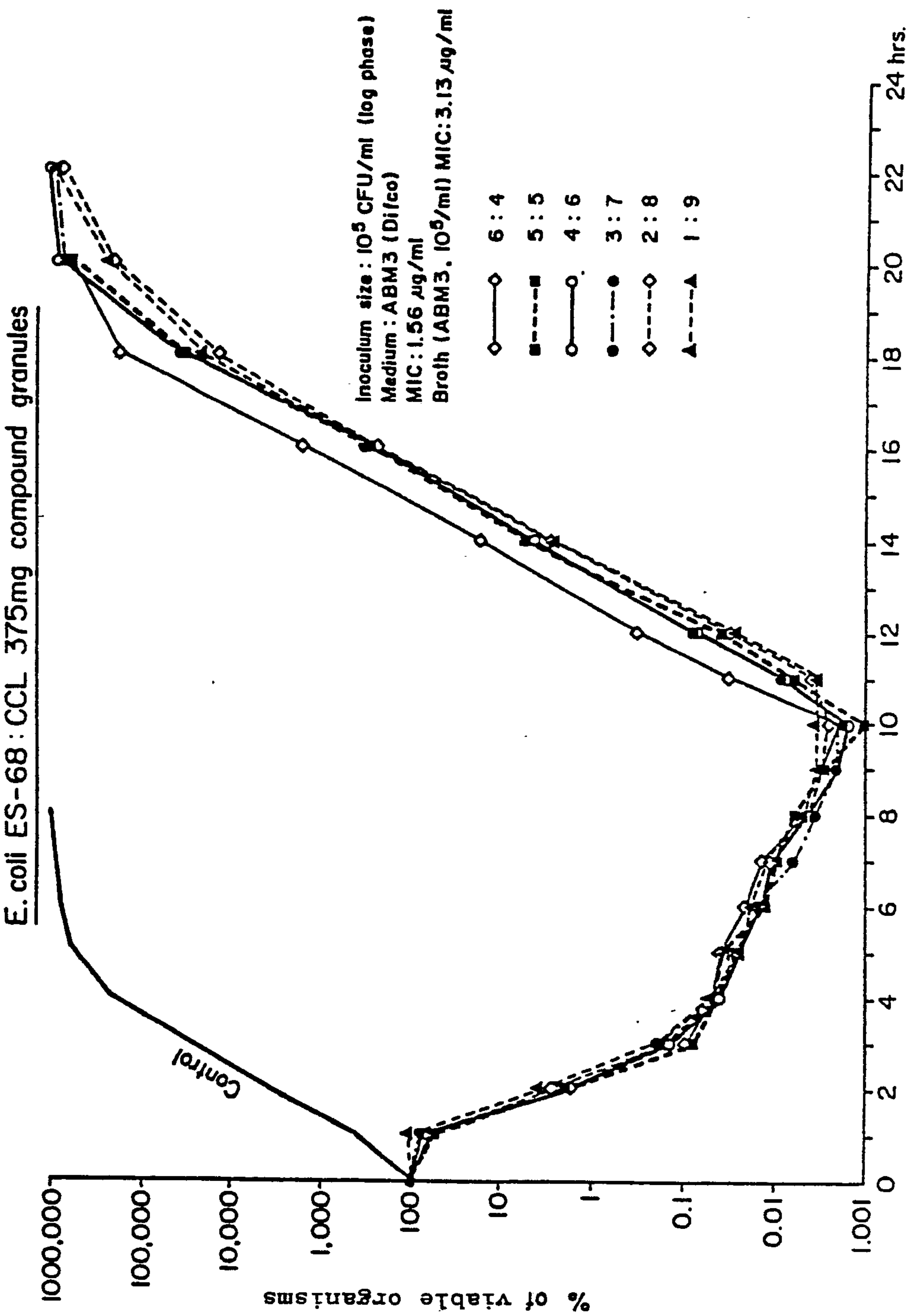

FIG. 4e.

E. coli ES-69 : CCL 375mg compound granules